# EUROPEAN PATENT SPECIFICATION

(11) **EP 0 708 635 B1**
(45) Date of publication and mention of the grant of the patent: **23.09.1998**
(21) Application number: 94919672.9
(22) Date of filing: 29.06.1994
(51) Int. Cl.: A61K 7/16

(54) **ORAL EFFERVESCENT COMPOSITIONS**
ORALE BRAUSEZUSAMMENSETZUNGEN
COMPOSITIONS ORALES EFFERVESCENTES

(30) Priority: 15.07.1993 EP 93305583
(43) Date of publication of application: 01.05.1996
(73) Proprietor: UNILEVER PLC, London EC4P 4BQ (GB); UNILEVER N.V., 3013 AL Rotterdam (NL)
(72) Inventor: WATERFIELD, Philip Christopher, Wirral, Merseyside L63 3DJ (GB); RAVEN, Stephen John, Makati, Metro Manila (PH)
(74) Representative: van Gent, Jan Paulus
(86) International application number: EP9402131
(87) International publication number: WO9502392

(56) References cited:
- WO-A-92/07550
- FR-A- 2 518 960
- US-A- 3 729 553
- US-A- 4 528 180

## Description

The present invention relates to toothpaste compositions which contain ingredients which upon contact with each other will produce an effervescent effect.

More particularly, the present invention relates to toothpaste compositions which consist of a first composition containing one of the ingredients necessary to produce an effervescent effect, and a second composition containing the other ingredient necessary to produce the effervescent effect.

Oral products consisting of a first composition and a second composition, each composition containing separated reactive components which, when brought together develop effervescence are already known in the art. Thus, in GB-A-2,112,642 (Colgate), a dual-compartment dentifrice dispensing container is described which contains in one compartment a portion of the dentifrice containing as reactive component sodium bicarbonate, and in the other compartment another portion of the dentifrice containing as reactive component an acidic compound.

Upon dispensing the two dentifrice portions from the container through a single outlet orifice, the two portions are brought into intimate contact, and the sodium bicarbonate and acidic compound react with each other, thereby releasing carbon dioxide gas which causes the dentifrice to effervesce when used in the mouth.

The first portion of this dentifrice, that is the portion containing the sodium bicarbonate, contains at least about 15 % by weight of sodium bicarbonate, and preferably from 20-40 % by weight.

One of the drawbacks of sodium bicarbonate is its salty taste, and this is acknowledged in the above GB-A-2,112,642. Formulations according to this patent are stated to have an initial salty taste, which changed to a pleasingly sweeter flavour during brushing, caused by the effervescence.

We have now surprisingly found, that lowering the sodium bicarbonate level results in a significantly less salty taste, without any significant reduction of effervescence. This provides for a dentifrice with an improved consumer acceptability in that it combines the required effervescence with a significantly more pleasant, less salty taste. It is observed, that in US-A-3 729 553 two-composition type liquid mouthwash systems are described with a low level of bicarbonate and citric acid, but such systems can contain up to 2 % free carbonate without taste problems.

Consequently, in its broadest aspect the present invention relates to a toothpaste composition comprising a first composition which contains as essential ingredient an alkalimetal bicarbonate, and a second composition which contains as essential ingredient from 2-15 % by weight of an acidic compound, and is characterised in that the alkalimetal bicarbonate is present in the first composition in an amount of 3-10 % by weight of that composition. The present invention will hereunder be described in more detail.

The first composition contains from 3-10 % by weight of the alkalimetal bicarbonate. The alkalimetal bicarbonate can be sodium or potassium bicarbonate, or a mixture thereof. Alkalimetal sesquicarbonate can also be used. Preferred is sodium bicarbonate.

The first composition can be formulated into any convenient form, such as a paste or a gel. A preferred form is a paste. The paste is usually formulated to have a pH of between 8.0 and 10.0, preferably 8.5-9.5. This paste may contain other, conventional toothpaste ingredients provided they are compatible with the alkalimetal bicarbonate. Thus, the paste will usually contain an abrasive cleaning agent such as chalk, silicas, hydroxyapatites, dicalciumphosphate, metaphosphates and so on, usually in amounts between 5 and 60 % by weight.

Furthermore, the paste may contain humectants such as glycerol, sorbitol, propyleneglycol, polyethyleneglycol, xylitol, lactitol and so on.

Binders and thickeners such as sodium carboxymethylellulose, xanthan gum, gum arabic etc. may also be included, as well as synthetic polymers such as polyacrylates and carboxyvinyl polymers such as Carbopol®.

Bicarbonate-compatible flavours such as peppermint and spearmint oils may also be included, as well as preservatives, colouring agents, pH-adjusting agents, sweetening agents and so on.

Anti-bacterial agents such as Triclosan may also be included, as well as anti-caries agents such as sodium monofluorophosphate, sodium fluoride, sodium trimetaphosphate, calcium glycerophosphate, calcium lactate etc..

Furthermore, anticalculus agents such as alkalimetal pyrophosphates may be included, as well as condensed phosphates. Alkalimetal silicates may also be included as a processing aid.

Further optional ingredients are Vitamin E, enzymes, antibodies, bacteriocins, statherins, defensins, bacteriophages, casein and casein digests, antimicrobial peptides, enzyme inhibitors, antibacterial adhesion polymers, lectins, tissue respiratory factor.

Anionic, nonionic, zwitterionic and ampholytic surfactants may also be included, such as sodium laurylsulphate, cocamidopropylbetain and ethylene oxide/propylene oxide block copolymers, as well as lactobionamides and glycoside derived surfactants.

The second composition which contains the acidic compound can also be formulated in any suitable form, such as a paste or a gel. The gel form is preferred. It is usually formulated to a pH of between 1.0 and 5.0, preferably 1.5-4.5.

Suitable examples of acidic compounds which can be used in the present invention are organic and/or inorganic acids such as organoleptically acceptable acids such as malic acid, glycolic acid, adipic acid, tartronic acid, phosphocitric acid, glycerophosphoric acid, citric acid, succinic acid, tartaric acid, lactic acid, phosphoric acid, pyrophosphoric acid, boric acid and so on. Citric acid is the preferred acid. Acid anhydrides may also be used, e.g. citric acid anhydride. Acid salts of the above acids may also be used, such as mono- and dihydrogen alkalimetal orthophosphate, mono-, di-, and trihydrogen alkalimetal pyrophosphate, mono- and di-alkalimetal citrates.

In general, the amount of acid compound in the second composition ranges from 2-15 % and preferably from 3-10 % by weight. Naturally, the amount of acid compound should be correlated with the amount of bicarbonate in the first composition to provide the required effervescence. Usually, the amount of bicarbonate to the amount of acid compound will have a weight ratio of 10:1 to 1:2. Preferred ratios range from 4:1 to 2:1.

The second composition may, furthermore, comprise further optional ingredients such as peroxides such as hydrogen peroxide, stannous compounds such as stannous fluoride and stannous pyrophosphate, zinc compounds such as zinc citrate and zinc sulphate, spangolites and spangolite-like materials, zinc hydroxyapatites, zinc hydrotalcites and zinc hydrotalcite-like materials, zinc silicates, zinc-containing zeolites, copper compounds such as copper sulphate, silver compounds such as silver-containing zeolites, Triclosan, chlorhexidine, potassium chloride, potassium acetate, potassium nitrate, potassium citrate, potassium hydrogen citrate, strontium chloride, strontium acetate, strontium citrate, vitamin E, gelling agents such as xanthan gum, CMC, polyacrylates, carboxyvinyl polymers, surfactants such as anionic, cationic, nonionic, zwitterionic and ampholytic surfactants, humectants such as sorbitol, glycerol, xylitol, propyleneglycol, polyethylene glycol, abrasives such as silica, propellants such as carbon dioxide, oxygen, nitrogen and lower aliphatic hydrocarbons.

The first and second composition may be separately packed in individual suitable containers, from which the consumer can extrude suitable amounts just before mixing it onto the brush or in the mouth.

The compositions are, however, preferably packed separately in multicompartment containers from which the compositions can be extruded through a single orifice to facilitate the use by the consumer. Suitable examples of multicompartment containers are given in US Patents 5,038,963, 5,020,694, 4,964,539, 4,528,180 and 4,849,213.

Naturally, the composition of each formulation in such multicompartment container, as well as the amount of each composition in the compartments of the container should be correlated, so that upon extrusion the properly balanced amounts of each composition is extruded to provide the required effervescence.

The invention will further be illustrated by way of Example.

### Example 1

In a dual compartment piston-type dispenser as described in US Patent 5,020,694 a series of effervescent toothpaste formulations were packed. One compartment contained the bicarbonate-paste, and the other the-acid containing gels. The formulations of the paste and the gel are given below. These formulations were statistically designed for comparison purposes with formulations according to GB-A-2,112,642.

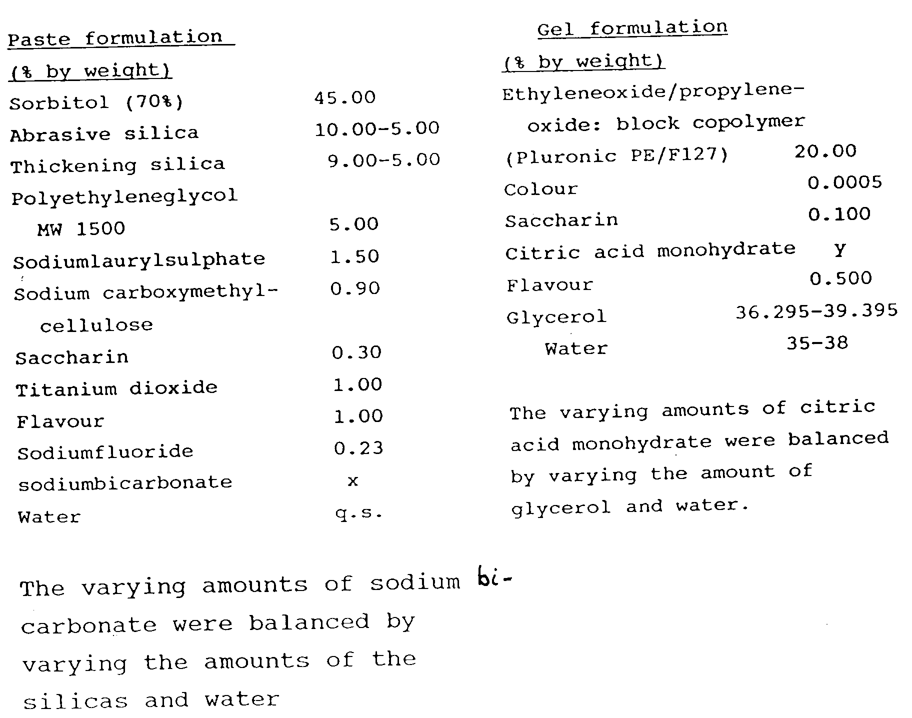

The products tested were coded 1-8, having the following values for x and y:

| Code | x (% by weight) | y (% by weight) |
|---|---|---|
| 1 | 5.2 | 2.9 |
| 2 (prior art) | 15.8 | 2.9 |
| 3 | 7.8 | 3.9 |
| 4 | 3 | 5 |
| 5 (prior art) | 18 | 5 |
| 6 | 5.2 | 7.1 |
| 7 (prior art) | 15.8 | 7.1 |
| 8 | 7.8 | 3.9 |

These products were tested by a panel of 12 experienced panellists for their sensory properties, in particular the flavour perception and the effervescence perception.These were ranked according to a scale of 1-10, ranging from extremely salty to no saltiness at all, and from very effervescent to not effervescent at all.

The following results were obtained:

| Code | Effervescence score | Saltiness score |
|---|---|---|
| 1 | 3.9 | 2.7 |
| 2 | 4.4 | 4.1 |
| 3 | 4.5 | 2.4 |
| 4 | 4.5 | 3.5 |
| 5 | 5.0 | 4.4 |
| 6 | 5.3 | 3.0 |
| 7 | 5.9 | 4.5 |
| 8 | 4.4 | 3.3 |

### Example 2

Products codes 8 and 5 were compared with each other by the above panel, as were products 1 and 2. They were assessed as to their effervescence and saltiness.

The following results were obtained:

| | Product 8 | Product 5 |
|---|---|---|
| Saltiness ranking | 2.44 (± 0.46) | 4.37 (± 0.46) |
| Effervescent ranking | 4.52 (± 0.47) | 5.03 (± 0.47) |

| | Product 1 | Product 2 |
|---|---|---|
| Saltiness ranking | 2.65 (± 0.46) | 4.13 (± 0.46) |
| Effervescent ranking | 3.85 (± 0.47) | 4.41 (± 0.47) |

### Example 3

Repeating Example 1, using tartaric or lactic acid instead of citric acid gives similar results, but the flavour quality is inferior to citric acid. Mixtures of 2 % citric acid and 1 % of either tartaric or lactic acid gives similar results too, but citric acid is perceived to have a superior flavour. Phosphoric acid also gives similar results but impairs foaming characteristics.

### Example 4

The following formulations are examples of a suitable first and second composition for use in the present invention.

| First Composition (% by weigth) | | Second Composition (% by weigth) |
|---|---|---|
| Sorbitol (70 %) | 45.00 | 45.00 |
| Abrasive silica | 10.00 | 12.00 |
| Thickening silica | 8.50 | 12.00 |
| Polyethyleneglycol MW 1500 | 5.00 | 5.00 |
| Sodium laurylsulphate | 1.50 | 1.50 |
| Sodium carboxy methylcellulose | 0.90 | 1.00 |
| Saccharin | 0.50 | 0.50 |
| Titanium dioxide | 1.00 | 1.00 |
| Sodium bicarbonate | 9.00 | -- |
| Flavour | 1.00 | 1.00 |
| Sodium fluoride | 0.23 | -- |
| Citric acid | -- | 4.57 |
| Water | q.s. | q.s. |

## Claims

1. An effervescent toothpaste composition with improved sensory properties, comprising a first composition containing as an essential ingredient an alkalimetal bicarbonate, and a second composition containing as essential ingredient from 2 - 15 % by weight of an acidic compound, characterised in that the alkalimetal bicarbonate is present in the first composition in an amount of 3 - 10% by weight of the first composition.

2. A composition according to claim 1, characterised in that the acidic compound is citric acid.

3. A composition according to claim 1 or 2, characterised in that the weight ratio of the alkalimetal bicarbonate to the acidic compound ranges from 10:1 to 1:2.

4. A composition according to claims 1-3, characterised in that the first and the second composition are separately packed in a multicompartment container having a single outlet orifice.

## Patentansprüche

1. Aufschäumende Zahnpastazusammensetzung mit verbesserten sensorischen Eigenschaften umfassend eine erste Zusammensetzung, die als wesentlichen Inhaltsstoff ein Alkalibicarbonat enthält, und eine zweite Zusammensetzung, die als wesentlichen Inhaltsstoff 2 bis 15 Gew.-% einer sauren Verbindung enthält, dadurch gekennzeichnet, daß das Alkalibicarbonat in der ersten Zusammensetzung in einer Menge von 3 bis 10 Gew.-% der ersten Zusammensetzung enthalten ist.

2. Zusammensetzung nach Anspruch 1, dadurch gekennzeichnet, daß die saure Verbindung Citronensäure ist.

3. Zusammensetzung nach Anspruch 1 oder Anspruch 2, dadurch gekennzeichnet, daß das Gewichtsverhältnis von Alkalibicarbonat zu saurer Verbindung im Bereich von 10:1 bis 1:2 liegt.

4. Zusammensetzung nach einem der Ansprüche 1 bis 3, dadurch gekennzeichnet, daß die erste und die zweite Zusammensetzung getrennt in einem Mehrkammerbehälter verpackt sind mit einer einzigen Auslaßöffnung.

## Revendications

1. Composition dentifrice effervescente avec des propriétés sensorielles améliorées, comprenant une première composition contenant à titre d'ingrédient essentiel un bicarbonate de métal alcalin et une seconde composition contenant à titre d'ingrédient essentiel de 2 à 15% en poids d'un composé acide, caractérisée en ce que le bicarbonate de métal alcalin est présent dans la première composition en une quantité de 3 à 10% en poids de la première composition.

2. Composition selon la revendication 1, caractérisée en ce que le composé acide est l'acide citrique.

3. Composition selon la revendication 1 ou 2, caractérisée en ce que le rapport pondéral du bicarbonate de métal alcalin au Composé acide est dans la gamme de 10:1 à 1:2.

4. Composition selon les revendications 1 à 3, caractérisée en ce que les première et seconde compositions sont conditionnées séparément dans un récipient à Compartiments multiples ayant un orifice de sortie unique.
